# EUROPÄISCHE PATENTANMELDUNG

(11) **EP 2 360 100 A1**
(43) Veröffentlichungstag der Anmeldung: **24.08.2011**
(21) Anmeldenummer: 10001657.5
(22) Anmeldetag: 18.02.2010
(51) Int. Cl.: B65B 57/00, B65B 59/00, G06Q 10/00, A61Q 13/00

(54) **Verfahren zum versandfertigen Herstellen individueller Kosmetikartikel und Vorrichtung**

(71) Anmelder: Spikker, Helmut, 59229 Ahlen (DE)
(72) Erfinder: Spikker, Helmut, 59229 Ahlen (DE)
(74) Vertreter: Bittner, Thomas L.

(57) **Zusammenfassung**

Die Erfindung betrifft ein Verfahren zum versandfertigen Herstellen individueller Kosmetikartikel umfassend die Schritte: Erfassen mehrerer Benutzereingaben, wobei für jede der mehreren Benutzereingaben Benutzerauswahlen aus der folgenden Gruppe erfasst werden: eine individuelle Zusammensetzung eines kosmetischen Stoffes, ein Behälter zum Aufnehmen des kosmetischen Stoffes, eine äußere Gestaltung des Behälter, eine Verpackung für den Behälter und äußere Gestaltung der Verpackung und aufeinander folgendes individuelles Herstellen und Verpacken der Kosmetikartikel den mehreren Benutzereingaben entsprechend, wobei für jeden der mehreren Kosmetikartikel gemäß den Benutzerauswahlen individuell die folgenden Schritte ausgeführt werden: Bereitstellen des Behälters zum Aufnehmen des kosmetischen Stoffes, Herstellen der äußeren Gestaltung des Behälters, Bereitstellen einer abzufüllenden Menge des kosmetischen Stoffes und Befüllen des Behälters mit der Menge des kosmetischen Stoffes, Verschließen des Behälters mit einem Verschluss, Verpacken des Behälters, Herstellen der äußeren Gestaltung der Verpackung und Ausgeben der Verpackung. Des weiteren betrifft die Erfindung eine Vorrichtung zum versandfertigen Herstellen individueller Kosmetikartikel.

## Beschreibung

Die Erfindung betrifft ein Verfahren zum versandfertigen Herstellen individueller Kosmetikartikel, insbesondere individueller Parfümartikel, sowie eine Vorrichtung.

### Hintergrund der Erfindung

Bei Kosmetikartikeln handelt es sich üblicherweise um Produkte, die in Massenfertigung hergestellt werden. Bekannte Herstellungsverfahren und Herstellungsanlagen sind auf eine solche Massenfertigung ausgerichtet und zielen auf eine Prozessoptimierung der Herstellung einer Vielzahl identischer Produkte ab.

Zunehmend besteht jedoch auch Bedarf für individualisierte Produkte auch auf dem Gebiet der Kosmetika. In diesem Zusammenhang besteht Bedarf für Technologien zu individualisierten Herstellung von Kosmetikartikeln.

### Zusammenfassung der Erfindung

Aufgabe der Erfindung ist es, ein Verfahren zum versandfertigen Herstellen individueller Kosmetikartikel sowie eine Vorrichtung zum Ausführen des Verfahrens bereitzustellen, mit denen individualisierte Kosmetikartikel effizient und zeitsparend hergestellt werden können.

Diese Aufgabe wird erfindungsgemäß durch ein Verfahren zum versandfertigen Herstellen individueller Kosmetikartikel nach dem unabhängigen Anspruch 1 sowie einer Vorrichtung nach dem unabhängigen Anspruch 12 gelöst. Vorteilhafte Ausgestaltungen der Erfindung sind Gegenstand von abhängigen Unteransprüchen.

Mit der Erfindung ist die Möglichkeit einer industriellen Fertigung individualisierter Kosmetikartikel geschaffen, die einzeln einer individuellen Nutzerauswahl entsprechend erzeugt sind. Bevorzugt finden die vorgeschlagenen Technologien Anwendung für die individuelle Herstellung von Parfümartikeln. Es ist dem Kunden ermöglicht, Merkmale des Kosmetikartikels seinen individuellen Wünschen entsprechend auszuwählen. Eingebunden ist die individualisierte Produktbestimmung in einen automatisierten Fertigungsprozess, sodass individuell gestaltete Kosmetikartikel in Masse hergestellt werden können. Es werden so die Vorteile personenspezifischer Kosmetikartikel einerseits und industrieller Fertigung andererseits beibehalten.

Das Erfassen der individuellen Benutzereingaben für die Ausgestaltung des Kosmetikartikels kann über ein lokales Terminal an der Herstellungs- und Verpackungsmaschine oder über ein mit einer Benutzerschnittstelle der Maschine verbundenes Terminal erfolgen. Beispielsweise kann vorgesehen sein, dass die Benutzerschnittstelle im Rahmen eines Clients bereitgestellt wird, welcher zum Erfassen der Benutzereingaben mit einem an die Herstellungs- und Verpackungsmaschine gekoppelten Server verbunden wird. In einer Ausführung erfolgt das Erfassen der Benutzereingaben über einen so genannten webbasierten Client, beispielsweise unter Nutzung des Internets. In dieser oder anderen Ausgestaltungen werden die Benutzereingaben über eine geeignete Benutzeroberfläche erfasst, die dem Benutzer mit bereitgestellten Menüs durch die Auswahl der zu erfassenden Benutzereingaben führt. Ein hierbei erstellter Datensatz mit den Informationen über die getroffene Benutzerauswahl wird dann an die zentrale Steuereinrichtung der Herstellungs- und Verpackungsmaschine übertragen.

Die vorgeschlagenen Technologien entfalten ihre Vorteile insbesondere im Zusammenhang mit der individualisierten Herstellung eines Parfüms. Hierbei kann vorgesehen sein, dass der Kunde oder Benutzer zunächst von einem Kundenberater Informationen über die individuelle Zusammenstellung eines Parfüms auf Basis vorhandener Duftstoffe erhält, insbesondere in einem Beratungsgespräch vor Ort. Ist nach dem Beratungsgespräch eine individuelle Zusammensetzung des Parfüms durch den Kunden getroffen, kann diese Auswahl über den Benutzerterminal erfasst werden. Zusätzlich zu der Zusammensetzung werden dann über den Benutzerterminal die weiteren Benutzerauswahlen getroffen. Die so erzeugten elektronischen Informationen werden dann an die Herstellungs- und Verpackungsmaschine übertragen, welche den Kosmetikartikel der Benutzerbestimmung entsprechend herstellt.

Eine bevorzugte Weiterbildung der Erfindung sieht vor, dass das Bereitstellen des Behälters zum Aufnehmen des kosmetischen Stoffes in der Behälterzuführeinheit einen Schritt zum Bereitstellen mittels mehrerer in paralleler Anordnung gebildeter Behälterspender umfasst, die jeweils mit unterschiedlichen Behälterformen befüllt werden. In Verbindung mit der Herstellung individualisierter Parfümartikel werden verschiedene Flakons bereitgestellt. Für die herzustellenden Kosmetikartikel wird dann der Benutzerauswahl entsprechend für jeden Artikel einzeln der entsprechende Flakon in den Herstellungsprozess einbezogen.

Bei einer zweckmäßigen Ausgestaltung der Erfindung kann vorgesehen sein, dass das Bereitstellen der abzufüllenden Menge des kosmetischen Stoffes einen Schritt zum Mischen einer oder mehrerer Basisstoffes mit einem oder mehreren Zusatzstoffen gemäß der Benutzerauswahl betreffend die individuelle Zusammensetzung umfasst. Bei der Ausführungsform zur Herstellung individueller Parfümartikel werden ein oder mehrere Basisduftstoffe mit einem oder mehreren Zusatzduftstoffen gemischt. Dieses erfolgt der individuellen Benutzerauswahl entsprechend. Insbesondere ist in diesem Zusammenhang vorgesehen, dass die Benutzerauswahl relative Anteile eines oder mehrerer Basisduftstoffe sowie relative Anteile eines oder mehrerer Zusatzduftstoffe festlegt, sodass ein individualisiertes Parfüm entsteht. Weiterhin betrifft die Benutzerauswahl auch die Gesamtmenge des Parfüms. Diesen Vorgaben entsprechend erfolgt die Bereitstellung der Parfümmenge in der Misch- und Befülleinheit, indem der ausgewählte Flakon befüllt wird.

Eine vorteilhafte Ausführungsform der Erfindung sieht vor, dass das Verfahren weiterhin das Erfassen einer Benutzertextauswahl bei der Benutzerauswahl betreffend die äußere Gestaltung des Behälters und das Herstellen einer Behälterbeschriftung mit der Benutzertextauswahl auf dem Behälter beim Herstellen der äußeren Gestaltung des Behälters umfasst. Der Benutzer erhält in diesem Verfahrensschritt die Möglichkeit, den Kosmetikartikel, insbesondere die Parfümflasche, mit selbst bestimmtem Text zu versehen. Beispielsweise wird zur äußeren Gestaltung des Behälters ein Etikett mit der Textauswahl des Benutzers hergestellt und dann auf die vom Benutzer ausgewählte Parfümflasche aufgeklebt. Die Etikettherstellung umfasst insbesondere das Bedrucken des Etiketts mit Hilfe eines Bedruckungssystems, welches zum Beispiel eine oder mehrere Tintenstrahldrucker aufweist.

Bevorzugt sieht eine Fortbildung der Erfindung vor, dass das Erfassen der Benutzertextauswahl weiterhin die folgende Schritte umfasst: Prüfen der Benutzertextauswahl auf unzulässigen Text und Unterbinden der Benutzertextauswahl, wenn beim Prüfen der Benutzertextauswahl festgestellt wird, dass die Benutzertextauswahl Text enthält, welcher gemäß vorab gespeicherter Prüfinformation als unzulässig klassifiziert ist. Auf diese Weise wird ausgeschlossen, dass unzulässige Beschriftungen hergestellt werden, zum Beispiel mit volks- oder persönlichkeitsverletzendem Inhalt. In einer Ausführungsform wird die Benutzertextauswahl zu diesem Zweck mit softwaretechnisch hinterlegten Texten verglichen, die unzulässige Inhalte darstellen. Es erfolgt auf diese Art und Weise eine Filterung der Benutzertextauswahl. Auch kann vorgesehen sein, dass beim Erfassen der Benutzertextauswahl dem Benutzer Textvorschläge am Benutzerterminal präsentiert werden, die dem Benutzer zur Auswahl bereitstehen. In einer Ausgestaltung des Verfahrens erfolgt für die Benutzertextauswahl eine Rechtschreibprüfung und -korrektur. Beim Erzeugen der elektronischen Informationen betreffend die erfassten Benutzereingaben wird die Benutzertextauswahl vorzugsweise dem so genannten UNI-Code entsprechend bereitgestellt. Auf diese Art und Weise wird eine sprachenunabhängige Zeichenkodierung erreicht, die dann in der Herstellungs- und Verpackungsmaschine verarbeitet wird.

Bei einer vorteilhaften Ausgestaltung der Erfindung kann vorgesehen sein, dass die erfasste Benutzertextauswahl in Bilddaten gewandelt wird, die als Teil der elektronischen Informationen von dem Benutzerterminal an die zentrale Steuereinrichtung der Herstellungs- und Verpackungsmaschine übertragen werden. Dieses ermöglicht die Bereitstellung der elektronischen Informationen für die Übertragung an die Herstellungs- und Verpackungsmaschine unabhängig von Schriftzeichen.

Eine Weiterbildung der Erfindung kann vorsehen, dass das Verschließen des Behälters mit dem Verschluss weiterhin die folgende Schritte umfasst: Bereitstellen einer Pumpeinrichtung, die konfiguriert ist, den kosmetischen Stoff mittels Handbetrieb aus Behälter zu fördern, Herstellen eines Förderschlauches an der Pumpeinrichtung der Größe des Behälters entsprechend und Anordnen der Pumpeinrichtung an dem Behälter. In Verbindung mit dem Herstellen eines individualisierten Parfümartikels wird nach der Auswahl einer für den vom Benutzer ausgewählten Behälter geeigneten Pumpeinrichtung ein Förderschlauch hergestellt, welcher hinsichtlich seiner Länge an die Pumpe und den ausgewählten Behälter angepasst ist. Für jeden einzelnen Kosmetikartikel wird der Förderschlauch individuell bereitgestellt. Die Pumpeneinrichtung wird zusammen mit dem Förderschlauch am Hals des Behälters angeordnet, wonach dann eine zugeordnete Kappe auf die Pumpeinrichtung aufgebracht wird. In der Kappe befindet sich auch ein Zerstäuber, um im Fall des individuellen Parfümartikels das Parfüm der Benutzerauswahl entsprechend auszubringen.

Eine bevorzugte Weiterbildung der Erfindung sieht vor, dass das Verfahren weiterhin die folgende Schritte umfasst: Erfassen einer weiteren Benutzertextauswahl bei der Benutzerauswahl betreffend die äußere Gestaltung der Verpackung und Herstellen einer Verpackungsbeschriftung mit der weiteren Benutzertextauswahl auf der Verpackung beim Herstellen der äußeren Gestaltung der Verpackung, wobei die Verpackungsbeschriftung mittels Beschriftungseinheiten für mehrere Verpackungen gleichzeitig erzeugt wird. Die Behandlung der Benutzertextauswahl in Verbindung mit der äußeren Gestaltung der Verpackung kann der oben beschriebenen Textbehandlung in Verbindung mit der Beschriftung auf dem Behälter entsprechend ausgeführt werden.

Bei einer zweckmäßigen Ausgestaltung der Erfindung kann vorgesehen sein, dass beim Herstellen der Verpackungsbeschriftung die Benutzertextauswahl mittels Laserbearbeitung direkt auf die Verpackung aufgebracht wird. Das Herstellen der Verpackungsbeschriftung erfolgt in einer Ausführungsform somit mittels direktem Lasern auf der Verpackungsoberfläche. Die Verpackungsgestaltungseinheit umfasst vorzugsweise mehrere Laserbeschriftungseinheiten, sodass mehrere Verpackungen gleichzeitig und / oder Vorder- und Rückseite gleichzeitig beschriftet werden können.

Eine vorteilhafte Ausführungsform der Erfindung sieht vor, dass das Verpacken des Behälters weiterhin das Bereitstellen einer zum Behälter passenden Innenverpackung und das Anordnen des befüllten Behälters in der Innenverpackung umfasst. Das Anordnen des Behälters kann vor oder nach dem Einlegen der passenden Innenverpackung, die auch als Inliner bezeichnet wird, in die Verpackung erfolgen.

Bevorzugt sieht eine Fortbildung der Erfindung vor, dass das Verfahren weiterhin die folgende Schritte umfasst: Erzeugen einer Artikelidentifikation für den individuellen Kosmetikartikel in der zentralen Steuereinrichtung, Zuordnen der Artikelidentifikation zu den elektronischer Informationen betreffend die jeweilige Benutzereingabe und Aufbringen der Artikelidentifikation auf die Verpackung beim Herstellen der äußeren Gestaltung der Verpackung. Die Artikelidentifikation, welche individuell für jeden der hergestellten Kosmetikartikel erzeugt wird und zum Beispiel mit alphanumerischen Zeichen gebildet ist, ermöglicht beispielsweise eine Identifikation des Artikels bei späterer Nachbestellung durch den Kunden. Der Artikelidentifikation zugeordnet werden die Benutzerauswahlen betreffend die individuelle Zusammensetzung des kosmetischen Stoffes, den Behälter zum Aufnehmen des kosmetischen Stoffes, die äußere Gestaltung des Behälters, die Verpackung für den Behälter sowie die äußere Gestaltung der Verpackung elektronisch gespeichert. Soll der individuelle Kosmetikartikel zu einem späteren Zeitpunkt hergestellt und verpackt werden, kann die zentrale Steuereinrichtung auf die so gespeicherten elektronischen Informationen zurückgreifen und den Herstellungsprozess erneut individuell durchführen.

Für Ausgestaltungen der Vorrichtung zum versandfertigen Herstellen individueller Kosmetikartikel gelten die im Zusammenhang mit gleichen Verfahrensausführungen genannten Erläuterungen entsprechend.

### Beschreibung bevorzugter Ausführungsbeispiele der Erfindung

Die Erfindung wird im Folgenden anhand von bevorzugten Ausführungsbeispielen unter Bezugnahme auf Figuren einer Zeichnung näher erläutert. Hierbei zeigen:
- Fig. 1: eine schematische Blockdarstellung einer Anordnung für ein Verfahren zum versandfertigen Herstellen individueller Kosmetikartikel,
- Fig. 2: eine schematische Blockdarstellung eines Anlagenteils einer Herstellungs- und Verpackungsmaschine zum versandfertigen Herstellen individueller Kosmetikartikel,
- Fig. 3: eine schematische Blockdarstellung eines weiteren Anlagenteils der Herstellungsund Verpackungsmaschine, welcher sich an den Anlagenteil in Fig. 2 anschließt,
- Fig. 4: einen vorderen Teil einer Behälterzuführeinheit von vorn,
- Fig. 5: einen hinteren Teil der Behälterzuführeinheit von vom,
- Fig. 6: eine Behältergestaltungseinheit von vom,
- Fig. 7: eine Misch- und Befülleinheit von vom,
- Fig. 8: eine Verschlusseinheit von vom,
- Fig. 9: eine Verpackungseinheit von vom und
- Fig. 10: eine Verpackungsgestaltungseinheit sowie ein nachgeschaltetes Aushebeband von vom.

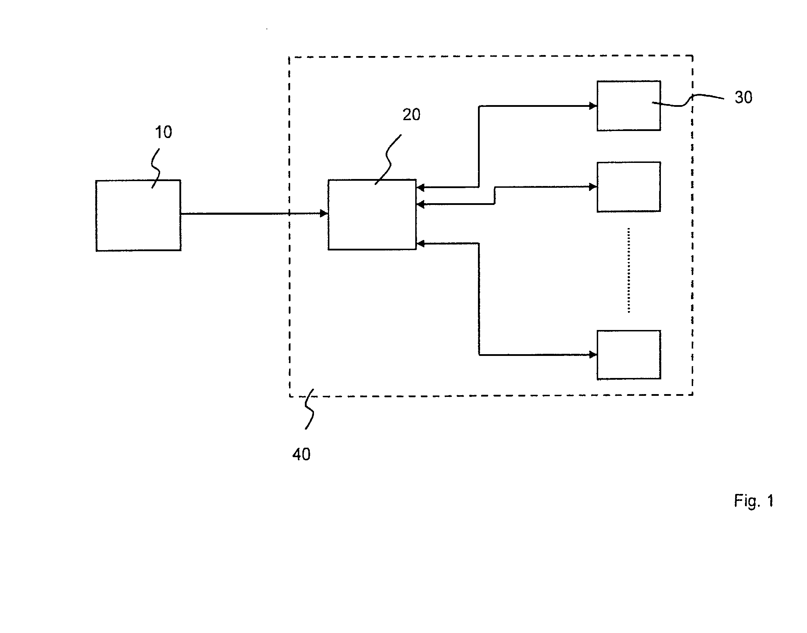

Fig. 1 zeigt eine schematische Blockdarstellung eines Systems zum versandfertigen Herstellen individueller Kosmetikartikel, insbesondere individueller Parfümartikel. Im Verfahren wird zunächst mit Hilfe eines Benutzerterminals 10 eine Benutzerauswahl für den herzustellenden Kosmetikartikel erfasst. Das Benutzerterminal 10 ist beispielsweise ein Personalcomputer, auf dem dem Benutzer mittels einer Applikationssoftware eine Benutzeroberfläche mit Menüsteuerung präsentiert wird, um die individuelle Benutzerauswahl(en) zu erfassen. In einer Ausführungsform werden über das Benutzerterminal 10 die folgenden Benutzereingaben erfasst: Eine individuelle stoffliche Zusammensetzung eines Parfüms, eine Behälterauswahl für einen Behälter zum Aufnehmen des Parfüms, eine äußere Gestaltung des Behälters, eine Verpackung für den Behälter und eine äußere Gestaltung der Verpackung. Wahlweise können weitere Benutzereingaben das Produkt betreffend erfasst werden.

Zusätzlich kann so vorgesehen sein, dass auch eine Benutzerauswahl für eine Pumpeinrichtung des Parfümartikels erfasst wird. In Verbindung mit der Benutzerauswahl betreffend die individuelle stoffliche Zusammensetzung des Parfüms werden Benutzereingaben betreffend ein oder mehrere Basisduftstoffe sowie ein oder mehrere Zusatzduftstoffe erfasst. Auf diese Weise wird ein individuelles Parfüm zusammengestellt. Der Benutzer kann diese Auswahl zuvor in einem Beratungsgespräch getroffen haben, in welchem er von einer geschulten Person hinsichtlich der Duftstoffoptionen sowie deren Zusammenmischung informiert wird. Die geschulte Person kann hierbei auch Probemischungen vor Ort durchführen, sodass der Kunde verschiedene Duftstoffvarianten prüfen kann. Die schließlich getroffene Auswahl hinsichtlich der individuellen Duftstoffzusammensetzung wird dann über das Benutzerterminal 10 erfasst.

Für die Auswahl des Behälters (Flakon) für das individuelle Parfüm werden dem Benutzer auf einer Anzeigefläche des Benutzerterminals 10 verschiedene Optionen angezeigt, aus denen der Benutzer auswählen kann. Gleiches gilt für die individuelle Auswahl einer Verpackung zur Aufnahme der Parfümflasche. Weiterhin werden über das Benutzerterminal 10 Benutzereingaben betreffend die äußere Gestaltung der Parfümflasche und der Verpackung erfasst. Hierbei kann vorgesehen sein, dass dem Benutzer Bild- und / oder Schriftelemente zur Auswahl bereitgestellt werden, unter denen der Benutzer auswählen kann. Darüber hinaus oder alternativ ist vorgesehen, dass der Benutzer individuelle Texteingaben machen kann für die Beschriftung der Parfümflasche und / oder der Verpackung.

In Verbindung mit der Benutzertextauswahl werden individuelle Textangaben des Benutzers nach der Eingabe überprüft, derart, dass insbesondere unzulässige Texte gesperrt werden. Der Benutzer wird dann darauf hingewiesen, dass die unzulässigen Texte nicht für die Beschriftung der Parfümflasche oder der Verpackung zur Verfügung stehen. Der Benutzer wird aufgefordert, eine andere Beschriftung anzugeben. Auch ist in einer Ausführungsform vorgesehen, dass die Texteingabe des Benutzers auf Rechtschreibung geprüft wird. Wahlweise können weitere Text- und / oder Bildfilter zur Anwendung kommen.

Erfasst werden darüber hinaus Angaben zum Benutzer, insbesondere betreffend eine Rechnungsadresse und eine Versandadresse, die auch identisch sein können. Ausgehend von den erfassten Benutzereingaben wird dann ein elektronischer Datensatz erzeugt, welcher Informationen über die Benutzereingaben umfasst. Der elektronische Datensatz wird gemäß Fig. 1 an eine zentrale Steuereinrichtung 20 einer Herstellungs- und Verpackungsmaschine 40 übermittelt. Die datentechnische Implementierung des Benutzerterminals 10 kann im Rahmen einer so genannten Client-Server-Anordnung erfolgen. Beispielsweise wird die Benutzeroberfläche zum Erfassen der Benutzereingaben im Rahmen eines so genannten Web-Clients bereitgestellt. Zwischen dem Benutzerterminal 10 und der zentralen Steuereinrichtung 20 können weitere datentechnische Anlagen angeordnet sein, zum Beispiel weitere Servereinrichtungen. Die zentrale Steuereinrichtung 20 steuert ausgehend von den empfangenen Benutzereingaben Anlageneinheiten 30 der Herstellungs- und Verpackungsmaschine 40, sodass der vom Benutzer ausgewählte und definierte Kosmetikartikel, bei dem es sich im beschriebenen Ausführungbeispiel um ein individuelles Parfüm handelt, entsprechend hergestellt wird.

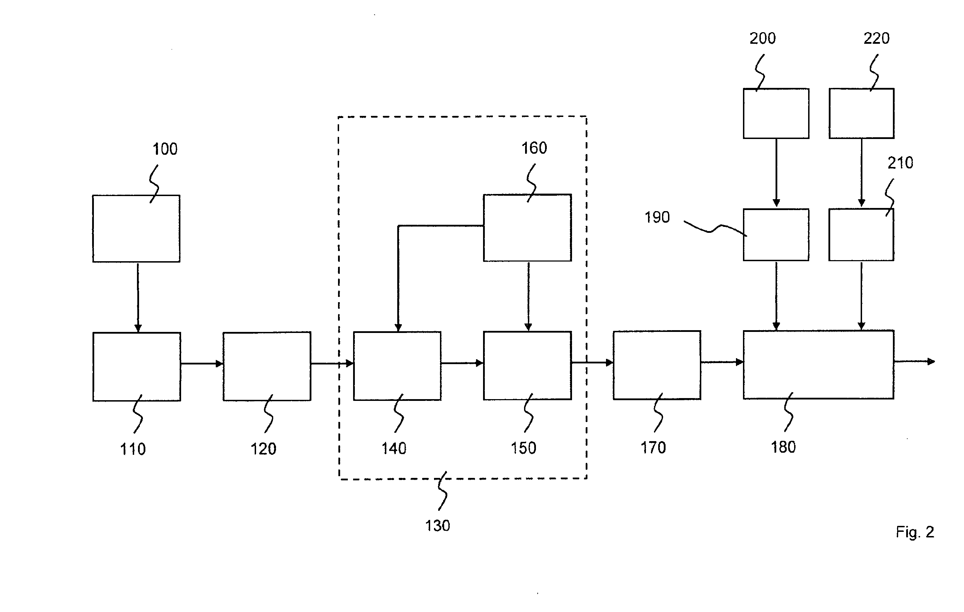

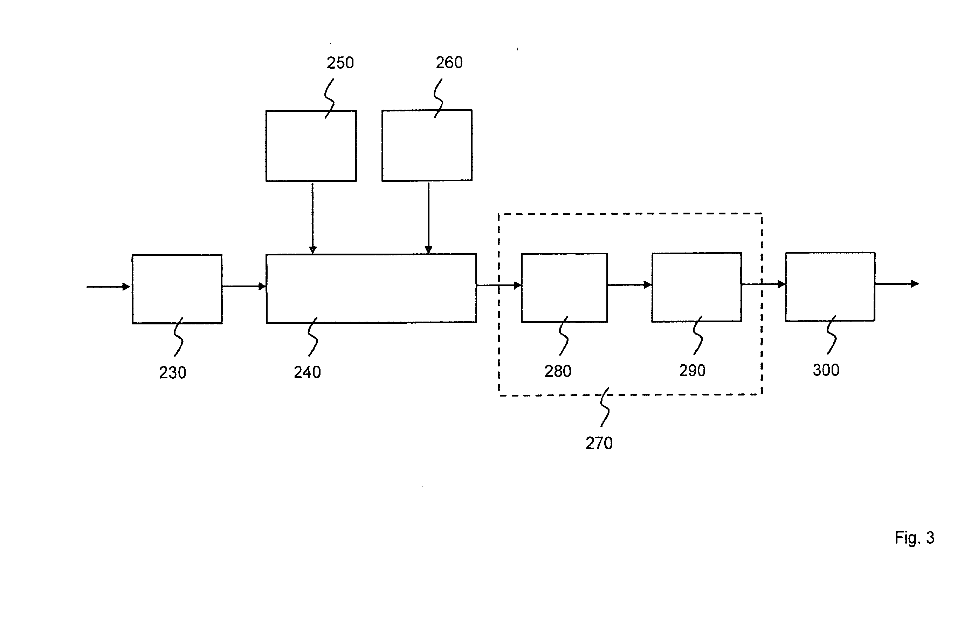

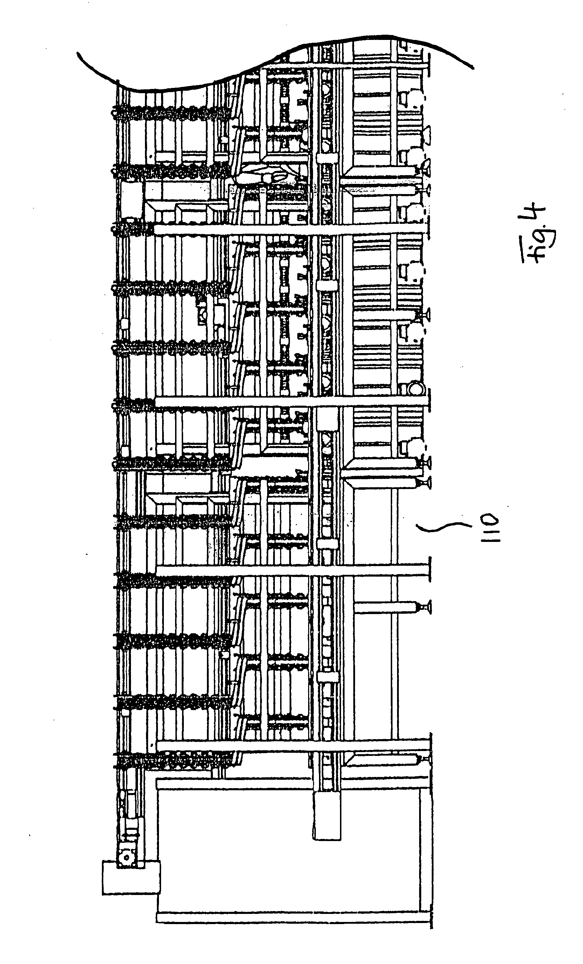

Der Herstellungsprozess wird im Folgenden unter Bezugnahme auf schematische Blockdarstellungen in den Fig. 2 und 3 für die Herstellungs- und Verpackungsmaschine 40 näher erläutert. Fig. 4 bis 10 zeigen Anlagenteile der Herstellungs- und Verpackungsmaschine 40 in einer Ausführungsform.

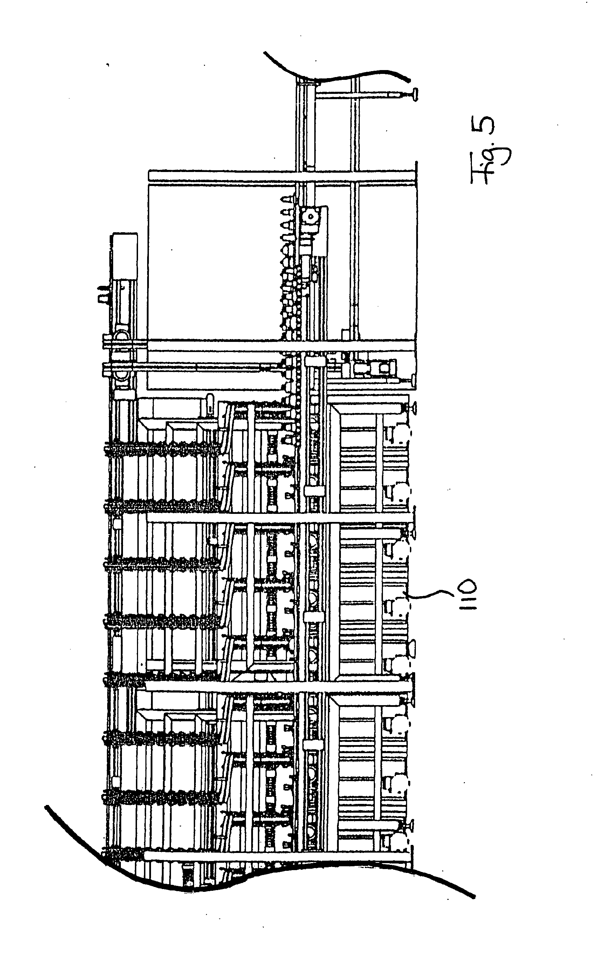

Die nachfolgend beschriebenen Schritte des Verfahrens zum versandfertigen Herstellen eines individuellen Kosmetikartikels in Form eines Parfüms werden den zuvor erfassten Benutzereingaben entsprechend ausgeführt. Aus einem Behälterspeicher 100 wird der Benutzerauswahl entsprechend eine Parfümflasche über eine Behälterzuführeinheit 110 bereitgestellt. Fig. 4 und 5 zeigen eine Darstellung mit einem vorderen und einem hinteren Teil der Behälterzuführeinheit 110 in einer Ausführungsform. Die Parfümflasche gelangt dann in einem Fertigungstransportbehälter oder -modul zu einem Modul 120 zur RFID-Produkt-Auftragskopplung. Hier wird ein am Fertigungstransportbehälter angebrachtes RFIF-Modul mit elektronischen Informationen betreffend die individuelle Herstellung des Artikels versehen. Diese elektronischen Informationen auf dem RFID-Chip werden im weiteren Herstellungsprozess an den einzelnen Maschinenstationen ausgelesen und zur individualisierten Fertigung herangezogen.

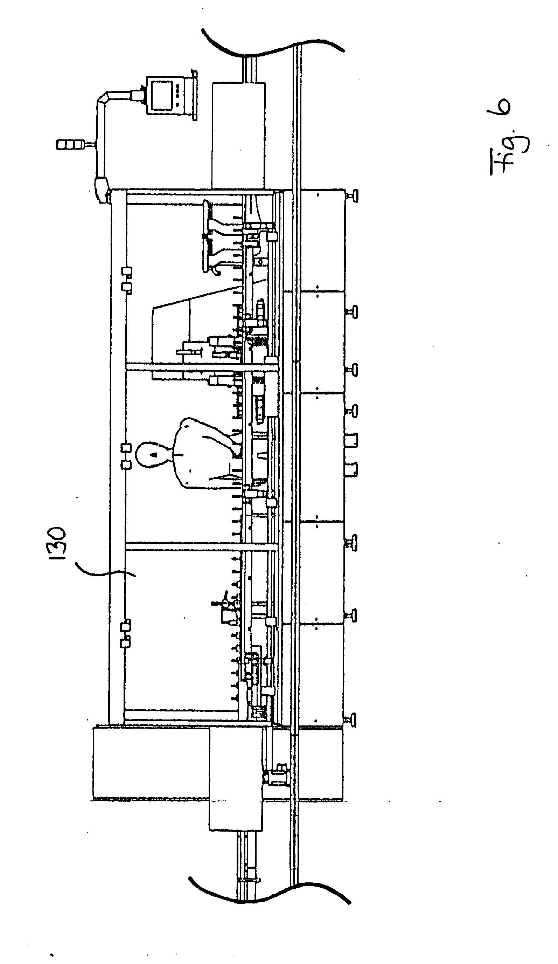

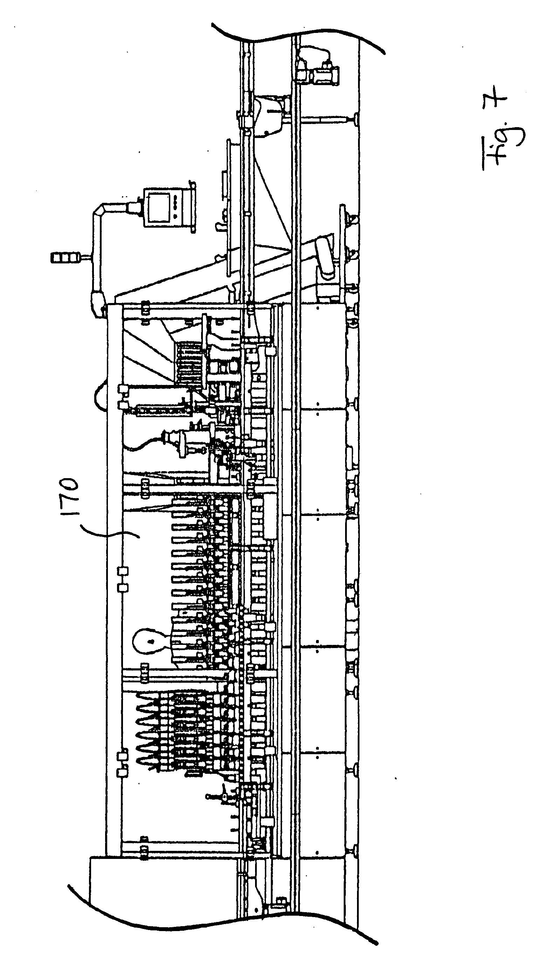

Eine Behältergestaltungseinheit 130 ist mit einem Bodenetikettmodul 140, einem Etikettmodul 150 sowie einem Etikettherstellungsmodul 160 gebildet. Fig. 6 zeigt eine Darstellung mit der Behältergestaltungseinheit 130 in einer Ausführungsform. Im Etikettherstellungsmodul 160 werden Klebeetiketten der Benutzerauswahl entsprechend hergestellt, indem Rohetiketten mittels eines Bedruckungssystems, welches zum Beispiel ein oder mehrere Tintenstrahldrucker umfasst, bedruckt werden. Die so hergestellten Etiketten werden dann mittels des Bodenetikettmoduls 140 und dem Etikettmodul 150 am Boden und auf der oder den Seite(n) der Parfümflasche aufgeklebt. Anschließend wird die Parfümflasche in einer Misch- und Befülleinheit 170 mit dem Parfüm befüllt, welches in seiner Zusammensetzung der Benutzerauswahl hinsichtlich des einen oder der mehreren Basisduftstoffe sowie des einen oder der mehreren Zusatzduftstoffe entspricht. Fig. 7 zeigt eine Darstellung mit der Misch- und Befülleinheit 170 in einer Ausführungsform.

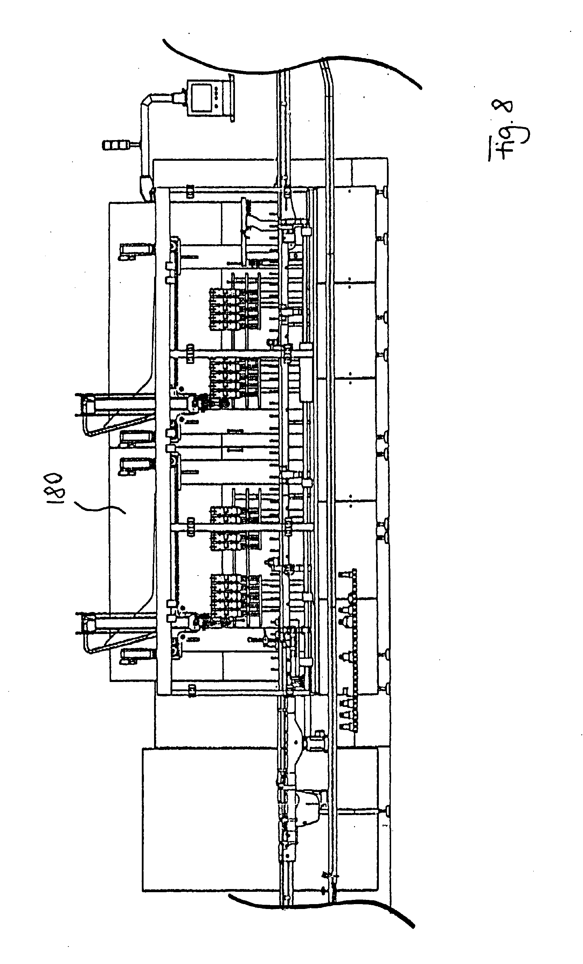

Von der Misch- und Befülleinheit 170 gelangt die nun mit einem oder mehreren Etiketten versehene und mit dem Parfüm befüllte Parfümflasche zu einer Verschlusseinheit 180, in welcher die Parfümflasche mit einer Sprühpumpe sowie einer zugehörigen Kappe versehen wird, nachdem zuvor ein an der Sprühpumpe angebrachter Sprühschlauch hinsichtlich seiner Länge der ausgewählten Parfümflasche entsprechend angepasst wurde, was insbesondere mittels einer Schlauchkürzung erfolgen kann. Fig. 8 zeigt eine Darstellung mit der Verschlusseinheit 180 in einer Ausführungsform. Die der Benutzerauswahl entsprechende Sprühpumpe sowie die zugehörige Kappe gelangen zu der Verschlusseinheit 180 über Zuführmodule 200, 220 sowie Sortiermodule 190, 210, die unterschiedlichen Pumpeinrichtungen zugeordnet sind.

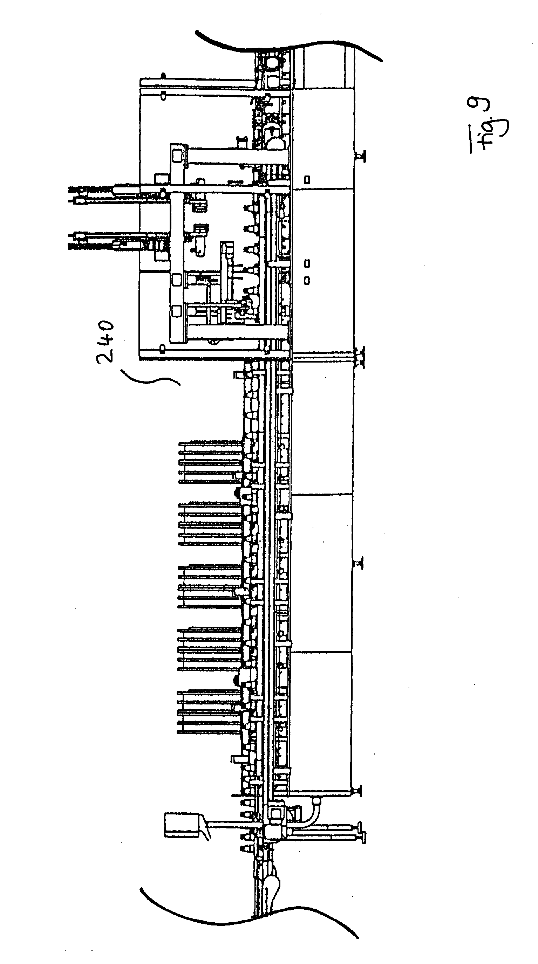

Der Herstellungsprozess setzt sich dann in dem in Fig. 3 dargestellten Anlagenabschnitt fort. In einem Kappenaufsatzmodul 230 wird die Parfümflasche der Benutzerauswahl entsprechend mit einer ausgewählten Kappe versehen. In einer Verpackungseinheit 240, in welcher über eine Faltschachtelbereitstellungseinheit 240 eine der Benutzerauswahl entsprechende Verpackung bereitgestellt wird, erfolgt das Einlagern der Parfümflasche in die Verpackung. Zu diesem Zweck steht die Verpackungseinheit 250 weiter in Verbindung mit einer Zuführeinrichtung 260 für so genannte Inliner, die als der ausgewählten Parfümflasche entsprechende Verpackungseinlagen dienen. Fig. 9 zeigt eine Darstellung mit der Verpackungseinheit 240 in einer Ausführungsform. Die befüllte und verschlossene Parfümflasche wird in eine an die äußere Flaschenform angepasste Verpackungseinlage eingelegt, um dann Verpackungseinlage und hieran angeordnete Flasche in der Verpackung anzuordnen.

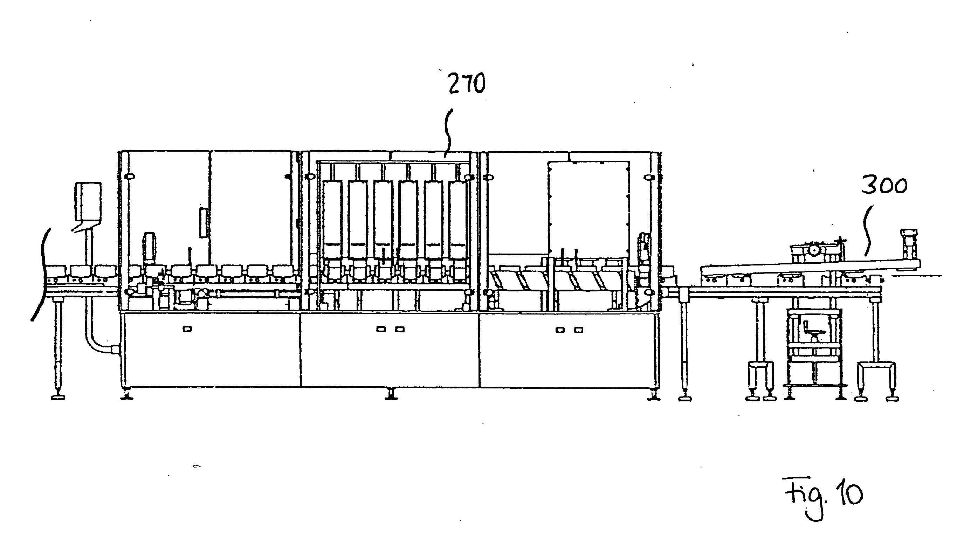

Danach wird die Verpackung in einer nachgeschalteten Verpackungsgestaltungseinheit 270 der Benutzerauswahl entsprechend von Außen gestaltet. Hierzu verfügt die Verpackungsgestaltungseinheit 270 über Lasermodule 280, 290 zur Beschriftung der Verpackung auf der Vorder- und der Rückseite. Bevorzugt wird die vom Benutzer ausgewählte äußere Gestaltung der Verpackung, insbesondere betreffend einen Beschriftungstext, direkt auf die äußere Oberfläche der Verpackung aufgelasert. Hierbei ist im Rahmen einer Parallelisierung von Verfahrensschritten vorgesehen, mit Hilfe mehrerer Lasereinheiten mehrere Verpackungen gleichzeitig individuell zu beschriften und zu gestalten. Der so hergestellte Parfümartikel wird dann über ein Aushebeband 300 geführt und hierdurch ausgegeben. Fig. 10 zeigt eine Darstellung mit der Verpackungsgestaltungseinheit 270 und dem Aushebeband 300 in einer Ausführungsform.

Dem vorangehend beschriebenen Ausführungsbeispiel entsprechend wird jeder Parfümartikel, welcher mit der Herstellungs- und Verpackungsmaschine 40 hergestellt wird, individuell der zugeordneten Benutzerauswahl entsprechend erzeugt. Für jeden Artikel werden die Einheiten oder Module der Herstellungs- und Verpackungsmaschine 40 individuell angesteuert, um so den vom Benutzer definierten Artikel individualisiert herzustellen. Es werden die Vorteile einer individuellen Produktdefinition durch den Kunden mit dem industrialisierten Fertigungsprozess kombiniert. Die Herstellungsvorrichtung verfügt über ein hohes Maß an Flexibilität, um für jeden einzelnen Artikel bei der Herstellung die entsprechende Benutzerauswahl umzusetzen. Gleichzeitig erfolgt die Herstellung jedoch vollständig automatisiert.

Die in der vorstehenden Beschreibung, den Ansprüchen und der Zeichnung offenbarten Merkmale der Erfindung können sowohl einzeln als auch in beliebiger Kombination für die Verwirklichung der Erfindung in ihren verschiedenen Ausführungen von Bedeutung sein.

### Bezugszeichenliste

- 10: Benutzerterminal
- 20: zentrale Steuereinrichtung
- 30: Anlageeinheiten
- 40: Herstellungs- und Verpackungsmaschine
- 100: Behälterspeicher
- 110: Behälterzuführeinheit
- 120: Modul zur RFID-Produkt-Auftragskopplung
- 130: Behältergestaltungseinheit
- 140: Bodenetikettmodul
- 150: Etikettmodul
- 160: Etikettherstellungsmodul
- 170: Misch- und Befülleinheit
- 180: Verschlusseinheit
- 190, 210: Sortiermodule
- 200, 220: Zuführmodule
- 230: Kappenaufsatzmodul
- 240: Faltschachtelbereitstellungseinheit
- 250: Verpackungseinheit
- 260: Zuführeinrichtung
- 270: Verpackungsgestaltungseinheit
- 280, 290: Lasermodule
- 300: Aushebewand

## Patentansprüche

1. Verfahren zum versandfertigen Herstellen individueller Kosmetikartikel, insbesondere individueller Parfümartikel, wobei das Verfahren die folgenden Schritte umfasst:
- Erfassen mehrerer Benutzereingaben, denen jeweils unterschiedliche Benutzerauswahlen für einen individuellen Kosmetikartikel zugeordnet werden, mittels eines Benutzerterminals (10), wobei für jede der mehreren Benutzereingaben erfasst werden:
- eine Benutzerauswahl betreffend eine individuelle Zusammensetzung eines kosmetischen Stoffes,
- eine Benutzerauswahl betreffend einen Behälter zum Aufnehmen des kosmetischen Stoffes,
- eine Benutzerauswahl betreffend eine äußere Gestaltung des Behälter,
- eine Benutzerauswahl betreffend eine Verpackung für den Behälter und
- eine Benutzerauswahl betreffend eine äußere Gestaltung der Verpackung,
- Übertragen elektronischer Informationen betreffend die erfassten mehreren Benutzereingaben von dem Benutzerterminal (10) an eine zentrale Steuereinrichtung (20) einer Herstellungs- und Verpackungsmaschine (40) über eine elektronische Datenverbindung,
- Erzeugen von Maschinensteuerungsdaten unter Berücksichtigung der empfangenen elektronischen Informationen mittels der zentralen Steuereinrichtung (20),
- aufeinander folgendes individuelles Herstellen und Verpacken der Kosmetikartikel den mehreren Benutzerangaben entsprechend, indem die Herstellungs- und Verpackungsmaschine (40) gemäß den Maschinensteuerungsdaten gesteuert wird, wobei von Maschineneinheiten (30) der Herstellungs- und Verpackungsmaschine (40) für jeden der mehreren Kosmetikartikel individuell die folgenden Schritte ausgeführt werden:
- Bereitstellen des Behälters zum Aufnehmen des kosmetischen Stoffes gemäß der Benutzerauswahl betreffend den Behälter in einer Behälterzuführeinheit (110),
- Herstellen der äußeren Gestaltung des Behälters gemäß der Benutzerauswahl betreffend die äußere Gestaltung des Behälters in einer Behältergestaltungseinheit (130),
- Bereitstellen einer abzufüllenden Menge des kosmetischen Stoffes gemäß der Benutzerauswahl betreffend die individuelle Zusammensetzung und Befüllen des Behälters mit der Mange des kosmetischen Stoffes in einer Misch- und Befülleinheit (170),
- Verschließen des Behälters mit einem Verschluss in einer Verschlusseinheit (180, 230),
- Verpacken des Behälters gemäß der Benutzerauswahl betreffend die Verpackung des Behälters in einer Verpackungseinheit (240),
- Herstellen der äußeren Gestaltung der Verpackung gemäß der Benutzerauswahl betreffend die äußere Gestaltung der Verpackung in einer Verpackungsgestaltungseinheit (270) und
- Ausgeben der Verpackung über eine Ausgabeeinheit (300).

2. Verfahren nach Anspruch 1, wobei das Bereitstellen des Behälters zum Aufnehmen des kosmetischen Stoffes in der Behälterzuführeinheit (110) einen Schritt zum Bereitstellen mittels mehrerer in paralleler Anordnung gebildeter Behälterspender (100) umfasst, die jeweils mit unterschiedlichen Behälterformen befüllt werden.

3. Verfahren nach Anspruch 1 oder 2, wobei das Bereitstellen der abzufüllenden Menge des kosmetischen Stoffes einen Schritt zum Mischen einer oder mehrerer Basisstoffes mit einem oder mehreren Zusatzstoffen gemäß der Benutzerauswahl betreffend die individuelle Zusammensetzung umfasst.

4. Verfahren nach mindestens einem der vorangehenden Ansprüche, wobei das Verfahren weiterhin die folgenden Schritte umfasst:
- Erfassen einer Benutzertextauswahl bei der Benutzerauswahl betreffend die äußere Gestaltung des Behälters und
- Herstellen einer Behälterbeschriftung mit der Benutzertextauswahl auf dem Behälter beim Herstellen der äußeren Gestaltung des Behälters.

5. Verfahren nach Anspruch 4, wobei das Erfassen der Benutzertextauswahl weiterhin die folgenden Schritte umfasst:
- Prüfen der Benutzertextauswahl auf unzulässigen Text und
- Unterbinden der Benutzertextauswahl, wenn beim Prüfen der Benutzertextauswahl festgestellt wird, dass die Benutzertextauswahl Text enthält, welcher gemäß vorab gespeicherter Prüfinformation als unzulässig klassifiziert ist.

6. Verfahren nach Anspruch 4 oder 5, wobei die erfasste Benutzertextauswahl in Bilddaten gewandelt wird, die als Teil der elektronischen Informationen von dem Benutzerterminal an die zentrale Steuereinrichtung (20) der Herstellungs- und Verpackungsmaschine (40) übertragen werden.

7. Verfahren nach mindestens einem der vorangehenden Ansprüche, wobei das Verschließen des Behälters mit dem Verschluss weiterhin die folgenden Schritte umfasst:
- Bereitstellen einer Pumpeinrichtung, die konfiguriert ist, den kosmetischen Stoff mittels Handbetrieb aus Behälter zu fördern,
- Herstellen eines Förderschlauches an der Pumpeinrichtung der Größe des Behälters entsprechend und
- Anordnen der Pumpeinrichtung an dem Behälter.

8. Verfahren nach mindestens einem der vorangehenden Ansprüche, wobei das Verfahren weiterhin die folgenden Schritte umfasst:
- Erfassen einer weiteren Benutzertextauswahl bei der Benutzerauswahl betreffend die äußere Gestaltung der Verpackung und
- Herstellen einer Verpackungsbeschriftung mit der weiteren Benutzertextauswahl auf der Verpackung beim Herstellen der äußeren Gestaltung der Verpackung, wobei die Verpackungsbeschriftung mittels Beschriftungseinheiten für mehrere Verpackungen gleichzeitig erzeugt wird.

9. Verfahren nach Anspruch 8, **dadurch gekennzeichnet, dass** beim Herstellen der Verpackungsbeschriftung die Benutzertextauswahl mittels Laserbearbeitung direkt auf die Verpackung aufgebracht wird.

10. Verfahren nach mindestens einem der vorangehenden Ansprüche, wobei das Verpacken des Behälters weiterhin die folgenden Schritte umfasst:
- Bereitstellen einer zum Behälter passenden Innenverpackung und
- Anordnen des befüllten Behälters in der Innenverpackung.

11. Verfahren nach mindestens einem der vorangehenden Ansprüche, wobei das Verfahren weiterhin die folgenden Schritte umfasst:
- Erzeugen einer Artikelidentifikation für den individuellen Kosmetikartikel in der zentralen Steuereinrichtung,
- Zuordnen der Artikelidentifikation zu den elektronischer Informationen betreffend die jeweilige Benutzereingabe und
- Aufbringen der Artikelidentifikation auf die Verpackung beim Herstellen der äußeren Gestaltung der Verpackung.

12. Vorrichtung zum versandfertigen Herstellen individueller Kosmetikartikel, insbesondere individueller Parfümartikel, nach einem Verfahren nach mindestens einem der vorangehenden Ansprüche, mit:
- einem Benutzerterminal (10) zum Erfassen mehrerer Benutzereingaben, denen jeweils unterschiedliche Benutzerauswahlen für einen individuellen Kosmetikartikel zugeordnet werden, wobei das Benutzerterminal konfiguriert ist, für jede der mehreren Benutzereingaben zu erfassen:
- eine Benutzerauswahl betreffend eine individuelle Zusammensetzung eines kosmetischen Stoffes,
- eine Benutzerauswahl betreffend einen Behälter zum Aufnehmen des kosmetischen Stoffes,
- eine Benutzerauswahl betreffend eine äußere Gestaltung des Behälter,
- eine Benutzerauswahl betreffend eine Verpackung für den Behälter und
- eine Benutzerauswahl betreffend eine äußere Gestaltung der Verpackung, und
- einer Herstellungs- und Verpackungsmaschine (40) mit einer zentralen Steuerung (20) und
- einer elektronischen Datenverbindung zwischen dem Benutzerterminal (10) und der zentralen Steuereinrichtung (20), wobei die elektronische Datenverbindung konfiguriert ist zum Übertragen elektronischer Informationen betreffend die erfassten mehreren Benutzereingaben von dem Benutzerterminal (10) an die zentrale Steuereinrichtung (20),
wobei die zentrale Steuereinrichtung (20) konfiguriert ist, Maschinensteuerungsdaten unter Berücksichtigung der empfangenen elektronischen Informationen zu erzeugen, und wobei die Herstellungs- und Verpackungsmaschine (40) konfiguriert ist, Kosmetikartikel den mehreren Benutzerangaben entsprechend aufeinander folgend individuell herzustellen und zu verpacken, indem die Herstellungs- und Verpackungsmaschine (40) gemäß den Maschinensteuerungsdaten gesteuert wird und für jeden der mehreren Kosmetikartikel individuell die folgenden Schritte ausgeführt:
- Bereitstellen des Behälters zum Aufnehmen des kosmetischen Stoffes gemäß der Benutzerauswahl betreffend den Behälter in einer Behälterzuführeinheit (110),
- Herstellen der äußeren Gestaltung des Behälters gemäß der Benutzerauswahl betreffend die äußere Gestaltung des Behälters in einer Behältergestaltungseinheit (130),
- Bereitstellen einer abzufüllenden Menge des kosmetischen Stoffes gemäß der Benutzerauswahl betreffend die individuelle Zusammensetzung und Befüllen des Behälters mit der Mange des kosmetischen Stoffes in einer Misch- und Befülleinheit (170),
- Verschließen des Behälters mit einem Verschluss in einer Verschlusseinheit (180, 230),
- Verpacken des Behälters gemäß der Benutzerauswahl betreffend die Verpackung des Behälters in einer Verpackungseinheit (240),
- Herstellen der äußeren Gestaltung der Verpackung gemäß der Benutzerauswahl betreffend die äußere Gestaltung der Verpackung in einer Verpackungsgestaltungseinheit (270) und
- Ausgeben der Verpackung über eine Ausgabeeinheit (300).
